(19) Europäisches Patentamt
European Patent Office
Office européen des brevets

(11) Publication number: **0 358 660 B1**

(12) # EUROPEAN PATENT SPECIFICATION

(45) Date of publication of patent specification: **25.05.94**

(51) Int. Cl.⁵: **C07K 5/06**, C07K 5/08, C07K 5/10, A61K 37/02

(21) Application number: **88903584.6**

(22) Date of filing: **17.03.88**

(86) International application number: **PCT/US88/00879**

(87) International publication number: **WO 88/06890 (22.09.88 88/21)**

(54) **SYNTHETIC INHIBITORS OF MAMMALIAN COLLAGENASE.**

(30) Priority: **17.03.87 US 26933**

(43) Date of publication of application: **21.03.90 Bulletin 90/12**

(45) Publication of the grant of the patent: **25.05.94 Bulletin 94/21**

(84) Designated Contracting States: **AT BE CH DE FR GB IT LI LU NL SE**

(56) References cited:

| | |
|---|---|
| US-A- 4 113 715 | US-A- 4 146 611 |
| US-A- 4 154 946 | US-A- 4 297 275 |
| US-A- 4 371 465 | US-A- 4 371 466 |
| US-A- 4 382 081 | US-A- 4 500 467 |
| US-A- 4 560 506 | US-A- 4 595 700 |
| US-A- 4 611 002 | US-A- 4 681 966 |

(73) Proprietor: **RESEARCH CORPORATION TECHNOLOGIES, INC.**
**101 N. Wilmot Road, Suite 600**
**Tucson Arizona 85711(US)**

(72) Inventor: **GRAY, Robert, D.**
**225 Blankenbaker Lane**
**Louisville, KY 40207(US)**
Inventor: **SPATOLA, Arno, F.**
**906 Burning Springs Circle**
**Louisville, KY 40207(US)**
Inventor: **MILLER, Robert, B.**
**312 Abbey Lane**
**Vermon Hills, IL 60061(US)**

(74) Representative: **Brauns, Hans-Adolf, Dr. rer. nat. et al**
**Hoffmann, Eitle & Partner**
**Patentanwälte**
**Postfach 81 04 20**
**D-81904 München (DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

Chemical Abstracts; Vol. 104, no. 21, 179824n, issued 1986 (Columbus, Ohio, U.S.A.), R. Miller, "Incorporation of Coordinating amide bond surrogates within mammalian collagenase inhibitors", Pept. Struct. Funct. , Proc. Am. Pept. Symp., 9th 1985, 815-18 (Eng.)

Chemical Abstracts, vol. 84, no. 17, 117624g, issued 1976, (Columbus,Ohio U.S.A.), E. Miller, "Cleavage of type II and III collagens with mammalian collagenase", Biochemistry 1976, 15(4), 787-92 (Eng.).

CHEMICAL ABSTRACTS, vol. 106, no. 1, 5th January 1987, page 287, abstract no. 15045z, Columbus, Ohio, US; R.D. gRAY et al.: "Inhibition of mammalian collagenases by thiolcontaining peptides"

CHEMICAL ABSTRACTS, vol. 105, no. 25, 22nd December 1986, page 338, abstract no. 221504f, Columbus, Ohio, US; V. DIVE et al.: "New thiol inhibitor of Achromobacter iophagus collagenase. Specificity of the enzyme's S3'subsite"

CHEMICAL ABSTRACTS, vol. 98, no. 5, 31st January 1983, page 313, abstract no. 30391u, Columbus, Ohio, US; H.MACARTNEY et al.: "Characterization of beta 1 anticollagenase from human plasma and its reaction with polymorphonuclear leukocyte collagenase by disulfide/thiol interchange"

CHEMICAL ABSTRACTS, vol. 107, no. 5, 3rd August 1987, page 74, abstract no. 33148k, Columbus, Ohio, US; C.B. CAPUTO et al.: "Proteoglycan degradation by a chondrocyte metalloprotease. Effects of synthetic protease inhibitors"

## Description

The present invention relates to novel synthetic peptides. More particularly, the invention relates to novel peptides which are useful as inhibitors of mammalian collagenase.

Collagenases are proteolytic enzymes which initiate the degradation of collagen in vertebrates. In addition to their normal function in metabolism of connective tissue and wound healing, these endoproteinases have been implicated in a number of pathological conditions such as joint destruction in rheumatoid arthritis, periodontal disease, cornea ulceration and possibly tumor metastasis.

The mechanism of action of mammalian collagenases on the molecular level is fairly well understood. Tissue collagenases hydrolyze a specific peptide bond at a single cleavage site on each of the three collagen chains of triple helical collagen. This cleavage site is contained within the amino acid sequence Pro-Gln-Gly-Leu-(Ile)-Ala-Gly-Gln-Arg, with cleavage occurring between glycine 775 and leucine or isoleucine 776, in Types I, II and III collagen, the predominant collagen in skin, bone, tendon, dentin, fascia and cartilage. The collagenases are metallopeptidases which contain an essential zinc at the active site. The zinc is assumed to function by interactions with the scissile carbonyl of the substrate, thus facilitating hydrolysis of the peptide bond.

Compounds which coordinate to the zinc active site have the ability to inhibit the activity of the collagenase. Because of the clinical importance and the desirability of being able to control these enzymes' activity, there has been a widespread effort to design compounds which are capable of interacting with the enzyme binding site and preventing the enzymes' action. Consequently, there exist a number of synthetic peptides and chemically similar compounds which are claimed to have at least some effect in inhibiting the activity of mammalian collagenases. Many of these synthetic peptides are constructed so as to mimic the natural amino acid sequence flanking the collagenase cleavage site. For example, U.S. Patent No. 4,511,504 describes a number of carboxyalkyl peptide derivatives said to have inhibitory activity. U.S. Patent No. 4,263,293 relates to heterocyclic-containing amide compounds, U.S. Patent No. 4,235,885 discloses mercaptoacyl amino acid derivatives, U.S. Patent No. 4,327,111 teaches N-substituted mercaptoacyl propionamides, U.S. Patent No. 4,382,081 describes a wide variety of mercapto amino acid derivatives, all of which appear to have some level of collagenase inhibitory activity. Similarly, U.S. Patent No. 4,374,765 refers to the use of acyl derivatives of the peptide Gly-L-Cys-Gly-L-Gln-L-Glu-$NH_2$. U.S. Patent No. 4,367,233 refers to thioglycolic acid derivatives, and U.S. Patent No. 4,361,574 teaches alkanoic acid derivatives which are useful collagenase inhibitors. European Patent Application No. 85870005.7 discloses thiopeptolide derivatives as inhibiting collagenase substrates.

In addition to patents, the scientific literature also contains references to many collagenase inhibiting compounds. Clark, et al. (Life Sciences 37: 575-578 (1985) refer to N[[5-chloro-2-benzo thiazolyl)-thiophenyl]acetyl]-L-cysteine, said to be a powerful mammalian collagenase inhibitor. Deleusse, et al. [Biochem Biophys. Res. Comm. 133: 483-490, 1985) also refer to an inhibitor N-[3-N-(benzyloxycarbonyl)-amino-1-(R)-carboxypropyl]-L-leucyl-O-methyl-L-tyrosine-N-methylamide. Gray, et al. (Biochem. Biophys. Res. Comm. 101: 1251-1258, 1981) disclose a number of thiol-containing analogues of the collagen cleavage site. Additional thiol-containing peptides are disclosed by Gray, et al. in J. Cell Biochem., 32: 71-77, 1986. Carboxyalkyl peptide analogues are described by Gray, et al. in Federation Proc. 44: 1431, 1985. Miller, et al. also disclose thiol-containing peptides in an abstract. [Fed. Proc. 45: 1859 (1986)].

US-A-4474799 discloses a method for inhibiting the degredation of enkephalins by administration of an enzyme inhibitor of the general formula:

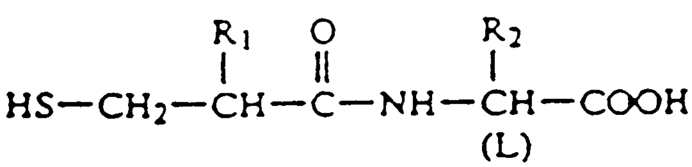

US-A-4297275, US-A-4595700 and Chemical Abstracts, vol. 106, no. 1, 1987, page 287, abstract no. 15045z disclose various mammalian collagenases which may include a moiety $HSCH_2$-CH(alkyl)-CO-AA-.. wherein AA may represent alanine, valine, leucine, isoleucine and O-methyl tyrosine.

Despite the large number of compounds showing inhibitory properties, the therapeutically useful commercially availably compounds are very few in number and are not altogether satisfactory in all respects for clinical use. Therefore, a continued need exists for an extremely potent and highly specific collagenase inhibitor which will have widespread therapeutic and commercial application. It has now been

discovered that a small class of novel thiol-containing peptides provides a level of collagenase inhibition not heretofore observed in the known inhibitory compounds.

The present invention relates to peptides of the formula:

$R_1SCH(R_2)CH(R_3)CO\text{-}AA_1[AA_2]_m[AA_3]_n\text{-}X$

wherein m is the integer 0 or 1; n is an integer from 0-2;

$AA_1$ is an amino acid selected from cyclohexylalanine, phenylalanine, naphthylalanine, tryptophan and tyrosine;

$AA_2$ is an amino acid selected from alanine, glycine, leucine, isoleucine and phenylalanine;

$AA_3$ is any amino acid;

$R_1$ is hydrogen, alkyl having from 1-10 carbon atoms, alkanoyl having from 2-10 carbon atoms, or aroyl having from 7-10 carbon atoms;

$R_2$ is hydrogen or alkyl having from 1-6 carbon atoms;

$R_3$ is hydrogen, alkyl having from 2-10 carbon atoms, cycloalkyl having from 3-6 carbon atoms, aryl or arylalkyl, wherein aryl moieties have from 6-10 carbon atoms;

X is $NH_2$, OH, $OCH_3$ or $OCH_2CH_3$;

and salts thereof,

with the proviso that if m is 0 and n is 1, then $AA_3$ is an amino acid selected from alanine, glycine, leucine, isoleucine and phenylalanine,

with the further proviso that if m is 0 and n is 2, then the amino acid $AA_3$ directly bonded to $AA_1$ is selected from alanine, glycine, leucine, isoleucine and phenylalanine, and

with the further proviso that if m and n are both 0, $R_2$ is H and $AA_1$ is phenylalanine, tryptophan or tyrosine, then X is not -OH.

The present invention also provides the use of the above compounds, including those excluded by the final proviso, for the manufacture of a medicament for the treatment of mammalian collagenase-related disorders.

In the formulation hereinabove, the group $R_1SCH(R_2)\text{-}CH(R_3)CO$, forms a peptide bond with the amino group of $AA_1$. Similarly, it is understood that whenever $AA_2$ or $AA_3$ are present, the various amino acids, $AA_1$, $AA_2$ and $AA_3$ are linked together by peptide bonds between the carboxy group of one amino acid moiety, and the amino gropu of the subsequent amino acid residue in the chain. For example, if in Formula I, m and n are both 1, then a peptide linkage is formed between the carboxy group of $AA_1$ and the amino group of $AA_2$ and another peptide is formed between the carboxy group of $AA_2$ and the amino group of $AA_3$.

The present invention also encompasses pharmaceutical compositions containing the aforementioned peptides as well as a method of treatment of collagenase-related disorders which comprises administration of an inhibitory effective amount of one or more of the claimed peptides.

The term "amino acid" as used herein refers to an organic acid whose molecule contains both a carboxyl group (COOH) and an amino group coupled with an alkyl, aryl or heterocyclic moiety. It will be understood that the term amino acid is intended to encompass both natural and synthetic residues; unsubstituted as well as mono or di-substituted natural amino acids, wherein the substitutes are halogen or lower alkyl containing 1 to 6 carbon atoms are encompassed by the term amino acids. Moreover, it is contemplated that n-formyl tryptophan may be employed in any position where a tryptophan residue is called for. The preferred amino acids contemplated in the present invention are the $\alpha$-amino acids. The preferred halogen substituent is chloro and the preferred alkyl substituent is methyl.

The following abbreviations for amino acids will be used throughtout the specification and claims:

| | | | |
|---|---|---|---|
| Ala | Alanine | Thr | Threonine |
| Gly | Glycine | Cys | Cysteine |
| Nal | Naphthylalanine | Met | Methionine |
| Leu | Leucine | Pro | Proline |
| Ile | Isoleucine | Lys | Lysine |
| Ser | Serine | Arg | Arginine |
| Asp | Aspartic Acid | Asn | Asparagine |
| Glu | Glutamic Acid | Gln | Glutamine |
| Phe | Phenylalanine | Tyr | Tyrosine |
| Trp | Tryptophan | | |

The peptides of the present invention represent inhibitory, thiol-containing analogues of the carboxyl side of the natural cleavage site of the collagen molecule. These novel peptides exhibit a very high affinity for this binding site of collagenase. The specificity and inhibitory activity of these compounds is greater than that observed with any commerically available collagenase inhibitors. A particularly surprising feature of the present peptides is the fact that the amino acid adjacent to the metal coordinating functionality, i.e. the thiol group, should preferably be a hydrophobic amino acid. This is a departure from the arrangement of the natural cleavage site in which alanine, an aliphatic neutral amino acid, occupies the corresponding position relative to the scissile carbonyl. Previously described synthetic peptide analogues have therefore tended to be constructed along the same lines, i.e., using a neutral amino acid such as leucine, isoleucine, alanine or glycine adjacent to the metal binding functionalities. It thus is particularly unexpected that not only does the use of a hydrophobic amino acid provide an active inhibitor, but it also provides a superior inhibitor.

The peptides of the present invention preferably may contain one, and up to four, amino acid residues. Additional amino acid residues may be present but do not add substantially to the activity of the product and simply serve to complicate the preparation of the peptide. The peptide structure is combined with a thiol-containing functional moiety which serves to bind to the zinc at the active site with the collagenase enzyme. The thiol-containing moiety in the final peptide has the formula:

$R_1SCH(R_2)CH(R_3)CO-$

wherein $R_1$ is hydrogen, alkyl, alkanoyl, or aroyl; $R_2$ is hydrogen or alkyl, and $R_3$ is hydrogen, alkyl, cycloalkyl, aryl or aralkyl. The alkanoyl moieties in the foregoing formula contain from 2-10 carbon atoms; the preferred alkanoyl moiety is acetyl. The aroyl substituents contain from 7-11 carbon atoms, with benzoyl being particularly preferred. Alkyl moieties contain from 2-10, and preferably from 2-6, carbon atoms and may be straight-chain or branched; isobutyl is the particularly preferred alkyl substituent. Aryl and the aryl in arylalkyl contain from 6-10 carbon atoms; the preferred aryl is phenyl. It will also be understood that the aryl moieties may be substituted with one, two or three substituents selected from the following alkyl, alkoxy, amino, hydroxy, or alkanoyloxy, the alkylalkoxy and alkanoyloxy moieties containing from 1-6 carbon atoms. Overall, the preferred thiol-containing moiety is one in which $R_1$ is hydrogen, $R_2$ is hydrogen or methyl and $R_3$ is alkyl, preferably isobutyl.

As noted above, one of the most essential elements of the peptide is the presence of a hydrophobic amino acid ($AA_1$) at the position one amino acid removed from the carbonyl functionality. In other words, besides the amino group and the carboxy group, $AA_1$ contains an hydrophobic residue, i.e., is nonpolar. For example, the hydrophobic residue includes but is not limited to an heterocyclic moiety containing 1, 2 or 3 ring heteroatoms selected from the group consisting of nitrogen, oxygen or sulfur in which the ring contains 5-10 ring atoms and 4-9 carbon ring atoms and which may be heteroaryl or partially or fully saturated, e.g., indolyl, (as in trypyophan); an aromatic moiety containing 6 to 10 ring carbon atoms; e.g., phenyl or $\alpha$ or $\beta$ -naphthyl, its alicyclic analogs which may be completely saturated or partially saturated e.g., cyclohexyl, and the like. However, the preferred $AA_1$ contain aromatic or heterocyclic groups. This amino acid may be selected from among the naturally occurring amino acids such as phenylalanine, tryptophan, or tyrosine, or may be a synthetic aromatic amino acid such as naphthylalanine. It is possible to construct a highly effective inhibitor with the presence of a single amino acid of this type, for example, the compounds 1 and 2 of Table 1.

The presence of a second amino acid is usually preferred and can increase the activity of the inhibitors substantially. The choice of residue at this position is also narrowly limited, however, if activity is to be maximized. The amino acid at this position is preferably selected from the group consisting of alanine,

glycine, leucine, isoleucine and phenylalanine. The presence of an alanyl residue at this position drastically increases the inhibitory capacity of the compounds, and therefore, this amino acid is particularly preferred. However, although activity is somewhat reduced, the remaining amino acids of this group may also occupy this position and still retain a significant level of inhibitory capacity.

The identity of additional amino acids, i.e. $AA_3$, if present, is not particularly critical to the activity of the inhibitors and therefore may be selected from any of the twenty amino acids, although the third amino acid is preferably glutamine, as this mimics the sequence adjacent to the cleavage site. As noted above, the length of the amino acid sequence is not particularly critical, and activity may be retained by the addition of up to as many as twenty or more amino acid residues. However, since the addition of several more residues does not significantly enhance the effectiveness of the compounds and substantially increases the difficulty of their preparation, it is preferred that the additional residues be limited to a maximum of two.

Any of the amino acids used in the present peptides may be either the D or the L form; although the use of the D form may in some positions reduce activity somewhat, it may in some circumstances be desirable to sacrifice some activity for increase in stability of the product.

The compounds of the present invention are relatively simple to prepare. Preparation of the appropriate thiol acid starting materials, which are generally acetyl- protected, is achieved by art recognized procedures; a thorough discussion of the method of preparation is found in U.S. Patent No. 4,235,885, the teachings of which are incorporated herein by reference. The peptides may be prepared by any of the wide range of known methods. Among the more commonly used techniques are coupling via the dicyclohexylcarbodiimide method, or the solid phase Merrifield synthesis, in which a protected amino acid is bound to a resin particle as an ester bond. Amino acids having functional groups such as tyrosine are generally protected with an easily removed blocking group, which are well known to the skilled artisan. Each of these techniques is equally suitable for the present purposes. The protected peptide is then coupled to the appropriate acetyl protected thiol, again by any of the typical coupling procedures referred to above. The compounds so produced may be purified by chromatography electrophoresis, or any other suitable means, and the acetyl protecting group removed by treatment with dilute $NH_4OH$ in nitrogen-flushed methanol.

Therefore, using the techniques discussed hereinabove, the compounds of the present invention can be prepared by art recognized techniques. For example, compounds of Formula I can be prepared by reacting an acylating derivative of the thiol acid of Formula II

$$R_1SCH(R_2)CH(R_3)COOH \qquad II$$

with the amino group of $AA_1$ in the following amino acids sequence of Formula III

$$AA_1(AA_2)_m(AA_3)_n\text{-}X \qquad III$$

under amide forming conditions. The coupling may be facilitated by the presence of a coupling reagent, such as dicyclohexylcarbodiimide or 1-Ethyl-3-(3-di-methylamino-isopropyl) carbodimiimide and the like. Protecting groups may also be used in order to minimize side reaction. A variety of protecting groups known in the art may be employed. Examples of many of these possible groups may be found in "Protective Groups in Organic Synthesis", by T.W. Green, John Wiley and Sons. For example, the thiol acid of Formula II may be acetyl protected. If desired, the protecting groups can be removed by art recognized techniques, as discussed in "Protective Groups in Organic Synthesis" discussed hereinabove.

The present invention is also intended to encompass salts of the claimed peptides. These compounds form basic salts with various organic and inorganic bases. Among the salts which may be prepared are ammonium, alkali metal salts, alkaline earth metal salts and salts with organic bases such as dicyclohexamine. In those peptides in which Arg is added, acid addition salts may also be prepared, particularly acetate or hydrochloride salts. Although for obvious reasons, pharmaceutically acceptable salts are preferred, but the invention is not limited to them since non-pharmaceutically acceptable salts may prove useful in isolating the compounds of the invention.

The compounds of the invention contain an asymmetric carbon atom (C-2), and therefore exist as diastereomeric pairs, which can be resolved by chromatography. The invention therefore includes both the R and S isomers which may be used in isolation or as a racemic mixture.

The compounds disclosed herein have been demonstrated to be highly effective inhibitors of mammalian collagenase activity as shown in Table 1. Many of the compounds are effective even in the nanomolar range, and all tested compounds have been proven effective in micromolar quantities. They may be thus efficiently employed in treatment of any mammalian disease in which collagenase has been implicated as a causative factor as noted above. Formulation of pharmaceutical compositions depends upon the nature of

the condition to be treated. For example, for rheumatoid arthritis treatment, intraarticular injection may be the preferred mode of administration; the peptides in this case or for any other type of parenteral administration, will generally be administered with a pharmaceutically acceptable carrier such as a sterile solution containing other solutes, for example, sufficient saline or glucose to make the solution isotonic. The peptides may also be formulated into tablets or capsules for oral administration in combination with stabilizers, excipients, carriers, preservatives, or flavors, as is typical in pharmaceutical practice. The typical dosage is between 10-500 mg/kg of body weight of the mammal being treated.

TABLE I

| | | $IC_{50}$ (uM)* | | |
|---|---|---|---|---|
| | | Fast Isomer | | Slow Isomer |
| | Cl | | | |
| 1. | $HSCH_2CH[CH_2CH(CH_3)_2]CO$-Phe-$NH_2$ | | 1 | |
| 2. | $HSCH_2CH[CH_2CH(CH_3)_2]CO$-Trp-$NH_2$ | 1 | | 2 |
| 3. | $HSCH_2CH[CH_2CH(CH_3)_2]CO$-Phe-Ala-$NH_2$ | 0.3 | | 0.04 |
| 4. | $HSCH_2CH[CH_2CH(CH_3)_2]CO$-Trp-Ala-$NH_2$ | | 0.05 | |
| 5. | $HSCH_2CH[CH_2CH(CH_3)_2]CO$-Phe-Leu-$NH_2$ | 10 | | 4 |
| 6. | $HSCH_2CH[CH_2CH(CH_3)_2]CO$-Phe-Phe-$NH_2$ | | 2 | |
| 7. | $HSCH_2CH[CH_2CH(CH_3)_2]CO$-Nal-Ala-$NH_2$ | | 0.03 | |

*$IC_{50}$ refers to the approximate concentration of compound giving 50% inhibition of collagen degradation in an in vitro assay system using pig synovial collagenase. Because C-2 (containing the isobutyl side chain) is asymmetric, the compounds exist as diastereomeric pairs which can be resolved by chromatography. Where an individual diastereomer has been assayed, the result for each is reported. In cases where the diastereomers have not been resolved, the $IC_{50}$ values were obtained with a mixture containing approximately equal amounts of the two. Since the absolute configuration at C-2 is not known, the diastereomers are identified as 'fast' or 'slow' by their relative elution time from a $C_{18}$ reversed phase chromatographic system under standardized conditions.

The compounds of the present invention and their method of preparation will be better understood by reference to the following non-limiting examples.

EXAMPLE 1

Preparation of $HSCH_2CH[CH_2CH(CH_3)]CO$-L-Phe-$NH_2$

1. 2-(R,S)-[(Acetylthio)methyl]-4-methylpentanoyl-L-phenylalanine amide. To a stirring solution of (±)-2-acetylthiomethyl-4-methylpentanoic acid (800mg, 3.92 mmol) in 10 ml dimethylformamide at 0°C was added 0.43 ml N-methyl-morpholine (3.92 mmol) followed by a solution of dicyclohexylcarbodiimide in 4 ml dichloromethane. The solution was stirred overnight at room temperature. Solvents were removed in vacuuo and the residue taken up in ethyl acetate and filtered. The filtrate was washed with solutions of 10% citric acid, 5% $NaHCO_3$, and 20% NaCl. After drying over $Na_2SO_4$, the solvent was removed by flash evaporation to give a solid which was triturated with pentane and filtered. The yield was 711 mg

(52%). Two spots (presumably the expected diasteomers) were observed on TLC (silica gel 60) in two solvent systems [$R_f$ 0.38, 0.28 (50% ethyl acetate-hexane); $R_f$ 0.48, 0.35 (diethylether)]. Gas chromatographic-mass spectral analysis of the mixture showed a molecular ion of 350.1617 ($C_{18}H_{26}N_2O_3S$ = 350.1664). The diastereomers were resolved by preparative $C_{18}$-reversed phase HPLC.

2. 2-[(R,S)-Mercaptomethyl]-4-methylpentanoyl-L-phenylalanine amide.The resolved diastereomers 1 and 2 were each dissolved in methanol, flushed with nitrogen for 15-30 minutes and treated with 0.1 volume of concentrated $NH_4OH$ for 30-60 minutes. The resulting deprotected thiol was precipitated by adding water, acidified with acetic acid, and the product recovered by lyophilization. For diastereomer 1: Anal. Calcd. for $C_{16}H_{24}N_2O_2S$ O.2$H_2O$: C, 61.58; H, 7.88; N, 8.98. Found: C, 61.46; H, 7.77; N, 9.08. For diastereomer 2: Anal. Calcd. for $C_{16}H_{24}N_2O_2S$ O.1$H_2O$: C, 61.94; H, 7.86; N, 9.03. Found: C, 62.04; H, 7.91; N, 8.94.

EXAMPLE 2

Preparation of $HSCH_2CH[CH_2CH(CH_3)]CO$-L-Phe-L-Ala-$NH_2$

1. t-Butyloxycarbonyl-L-phenylalanyl-L-alanine amide. L-Alanine amide hydrobromide (500 mg, 2.95 mmol), t-butyloxcarbonyl-L-phenylalanine N-hydroxysuccinimide ester (885 mg, 2.95 mmol), and 0.41 ml (2.95 mmol) triethylamine were dissolved in 15 ml acetonitrile-methanol (2:1, v:v). The mixture was stirred overnight at room temperature. The solvent was then removed under reduced pressure at 40 °C and the residue extracted into ethyl acetate. The extract was washed successively with saturated $NaHCO_3$, water, 10% citric acid, and water. The organic layer was dried with $Na_2SO_4$ and the solvent removed by flash evaporation. The dried product weighed 0.6 g (61%).

2. L-Phenylalanyl-L-alanine amide trifluoroacetate. The product from step 1 above was dissolved in 3 ml trifluoroacetic acid. After 30 min at room temperature, the resulting deprotected peptide was precipitated with dry ether. The precipitate was collected by filtration, triturated with ether and dried. The yield was 0.58 g (111%).

3. 2-(R,S)-[(Acetylthio)methyl]-4-methylpentanoyl-L-phenylalanyl-L-alanine amide. L-Phenylalanyl-L-alanine amide trifluoroacetate (500 mg, 1.43 mmol), 0.2 ml triethylamine (1.43 mmol), 293 mg (±)-2-[-(acetylthio)methyl]-4-methylpentanoic acid, and 320 mg (1.43 mmol) dicyclohexylcarbodiimide were dissolved in 10 ml of ice-cold acetonitrile-methanol (1:1, v:v). The reaction mixture was kept on ice overnight and its progress monitored at 210 nm by reversed phase HPLC using a $C_{18}$ column and a linear gradient of 0.1% $H_3PO_4$ and acetonitrile. In order to obtain complete reaction of the peptide, an additional 530 mg of the protected thiol and 375 mg of the carbodiimide were added over a 36 hour period. The reaction mixture was warmed to room temperature and the precipitate removed by filtration. The desired product peptide derivatives were purified by preparative $C_{18}$ reversed phase HPLC (0.1% trifluoroacetic acid/acetonitrile) and recovered by lyophilization (218 mg, 36%). The resulting mixture of diastereomers was separated into two components, designated diastereomer 1 and diastereomer 2, by reversed phase HPLC as above. Gas chromatographic-mass spectral analysis of 1 and 2 gave the same fragmentation pattern and showed molecular ions of 421.2043 and 421, respectively ($C_{21}H_{31}N_3O_4S$ = 421.2035).

4. 2-[(R,S)-Mercaptomethyl]-4-methylpentanoyl-L-phenylalanyl-L-alanineamide. The resolved diastereomers 1 and 2 were dissolved in 2 ml methanol, flushed with nitrogen for 15-30 minutes and treated with 0.2 ml concentrated $NH_4OH$ for 30-60 minutes. The resulting deprotected thiol was precipitated by adding water, acidified with acetic acid, and the product recovered by lyophilization. For diastereomer 1 (24 mg): TLC $R_f$ 0.31 ($CHCl_3$-MeOH, 10:1), 0.72 ($CHCl_3$-MeOH, 5:1), 0.92 (BuOH-acetic acid-$H_2O$, 4:1:1); amino acid analysis: Phe:Ala, 1:1.04; Anal. Calcd. for $C_{19}H_{29}N_3O_3S$ 1.4 $H_2O$: C, 56.38; H, 7.92; N, 10.38; S, 7.92. Found: C, 56.63; H, 7.55; N, 9.52; S, 8.18. For diastereomer 2 (80 mg): TLC $R_f$ 0.20 ($CHCl_3$-MeOH, 10:1), 0.67 ($CHCl_3$-MeOH, 5:1), 0.89 (BuOH-acetic acid-$H_2O$, 4:1:1); amino acid analysis; Phe:Ala, 1:0.86; Anal. Calcd for $C_{19}H_{29}N_3O_3S$ 1.9 $H_2O$; C, 55.15; H, 7.99; N, 10.16; S, 7.75. Found C, 55.40; H, 7.45; N, 9.95; S, 7.96.

EXAMPLE 3

Preparation of $HSCH_2CH[CH_2CH(CH_3)]CO$-L-Phe-L-Leu-$NH_2$

1. t-Butyloxycarbonyl-L-phenylalanyl-L-leucine amide. L-Leucine amide hydrochloride (500 mg, 2.99 mmol), t-butyloxycarbonyl-L-phenylalanine N-hydroxysuccinimde ester (1069 mg, 2.95 mmol), and 0.41

ml (2.95 mmol) triethylamine were dissolved in 10 ml acetonitrile-methanol (1:1, v:v). The mixture was stirred at room temperature overnight. The solvent was removed under reduced pressure at 40 °C and the residue extracted into ethyl acetate. The extract was washed successively with saturated $NaHCO_3$, water, 10% citric acid, and water. The organic layer was dried with $Na_2SO_4$ and the solvent removed by rotary evaporation as above. The dried product weighed 0.94 g (83.9%).

2. L-Phenylalanyl-L-leucine amide trifluoroacetate. The product from step 1 above was dissolved in 3 ml trifluoroacetic acid. After 30 min at room temperature, the product was precipitated with dry ether. The precipitate was collected by filtration, triturated with ether and dried. The yield was 0.94 g (108%).

3. 2-(R,S)-[(Acetylthio)methyl]-4-methylpentanoyl-L-phenylalanyl-L-leucine amide. L-Phenylalanyl-L-leucine amide trifluoroacetate (780 mg, 2.0 mmol), 0.28 ml triethylamine (2.0 mmol), 409 mg (±)-2-[-(acetylthio)methyl]-4-methylpentanoic acid, and 513 mg (2.0 mmol) dicyclohexylcarbodiimide were dissolved in 10 ml ice-cold acetonitrile-methanol (1:1,v:v). The reaction mixture was kept on ice overnight and its progress monitored at 210 nm by reversed phase HPLC using a $C_{18}$ column and a linear gradient of 0.1% $H_3PO_4$ and acetonitrile. In order to obtain complete reaction of the peptide, and additional 530 mg of the protected thiol and 375 mg of the carbodiimide were added over a 36 hour period. The reaction mixture was warmed to room temperature and the precipitate removed by filtration. The product peptide derivatives were purified by preparative $C_{18}$ reversed phase HPLC (0.1% trifluoroacetic acid/acetonitrile) and recovered by lyophylization (440 mg, 47.5%). The resulting mixture of diastereomers were separated into two components, designated diastereomer 1 and diastereomer 2, by reversed phase HPLC as described above.

4. 2-[(R,S)-Mercaptomethyl]-4-methylpentanoyl-L-phenylalanyl-L-leucineamide. Each of the diastereomers were dissolved in 5 ml methanol, flushed with nitrogen for 15-30 minutes and treated with 0.5 ml concentrated $NH_4OH$ for 30-60 minutes. The resulting deprotected thiol was precipitated by adding water, acidified with acetic acid, and the product recovered by lyophilization. For diastereomer 1 (175 mg): TLC $R_f$ 0.19 ($CHCl_3$-MeOH, 10:1), 0.69 ($CHCl_3$-MeOH, 5:1), 0.97 (BuOH-acetic acid-$H_2O$, 4:1:1); amino acid analysis; Phe:Leu, 1:0.98; Anal. Calcd. for $C_{22}H_{35}N_3O_3S$ 1.2 $H_2O$: C, 59.62; H, 8.51; N, 9.48; S, 7.23. Found: C, 59.66; H, 8.51; N, 9.89; S, 6.61. For diastereomer 2 (160 mg): TLC $R_f$ 0.16 ($CHCl_3$-MeOH, 10:1), 0.67 ($CHCl_3$-MeOH, 5:1), 0.97 (BuOH-acetic acid-$H_2O$, 4:1:1); amino acid analysis: Phe:Leu, 1:1.01; Anal. Calcd. for $C_{22}H_{35}N_3O_3S$ 0.1 $H_2O$: C, 62.41; H, 8.38; N, 9.92; S, 7.57. Found: C, 62.11; H, 8.19; N, 9.59; S, 7.94.

## EXAMPLE 4

The following example demonstrates the method of testing for inhibitory activity.

### Collagenase Assay

Collagenase activity was determined after electrophoretic separation of degraded from undegraded type I collagen by polyacrylamide gel electrophoresis and densitometry as follows.

Acid-soluble calf skin collagen (0.25 mg/ml, approximately 0.8 M) was incubated at 35 °C for 1 hr with pig synovial collagenase (0.04 g protein) in 0.05 M tris-HCL, 0.2 M NaCl, 0.25 M glucose, 5 mM $CaCl_2$, 10% dimethyl sulfoxide, pH 7.6 in a total reaction volume of 20 L. Inhibitors were dissolved in dimethyl sulfoxide and the sulfhydryl titer determined in stock solutions immediately prior to use by the colorimetric procedure of Ellman [Ellman, G. L., Arch. Biochem. Biophys. 82: 70-77 (1959)]. At the end of the reaction period, the reactions were stopped by placing on ice and 20 L sample dilution buffer was added [Laemmli, U.K., Nature (London) 227: 680-685 (1970)]. The samples were then placed in a boiling water bath for 2-5 minutes after which collagen degradation products were separated from undegraded collagen by sodium dodecyl sulfate-polyacrylamide electrophoresis according to the procedure of Laemmli [1970]. The electrophoretograms were fixed in isopropanol/acetic acid/ water (100:40:300) and stained with 1% Coomassie Blue R-250. The percentage of collagen alpha chains degraded was estimated by scanning densitometry and integration of peak areas [Welgus et al., J. Biol. Chem. 256: 9511-9515 (1981)].

A spectrophotometric method was also utilized in some cases to determine collagenase activity [Lindy, S. et al., European J. Biochem. 156: 1-4 (1986)]. The conditions were the same as given above except that the reaction volume was 200 L, the temperature was 37 °C and the enzyme concentration was 1.2 g protein/ml. Stock solutions of inhibitors were prepared in 1 mM acetic acid in ethanol and the sulfhydryl titer determined colorimetrically by the method of Ellman (1956). The reaction progress was monitored for 6-10 minutes by following the increase in absorbance at 227 nm that accompanies denaturation of the collagen fragments. Initial rates of collagen degradation were determined from the linear portion of the progress

curves.

The results of the collagenase assays for a number of the present peptides are found in Table 1.

EXAMPLE 5

2-(R,S)-[Mercaptomethyl]-4-methylpentanoyl-L-cyclohexyl-L-alanine amide

To a solution of (±) -2-acetylthiomethyl-4-methyl pentanoic acid (5 mmole) the hydrochloride of the cyclohexylalanine-alanine-$NH_2$ and triethylamine (0.07 ml, 5 mmol) and dried methylene chloride (5 ml) was added (gradually over thirty minutes) 1-ethyl-3-(3-dimethylaminoisopropyl) carbodiimide hydrochloride (0.958 g, 5 mmol). (cyclohexyl alanine is the aliphatic analog of phenyl alanine.) The reaction mixture was stirred for 1 hour at 0°C and then overnight at room temperature. The progress of the reaction was monitored by TLC. After completion of the reaction, ethyl acetate (50 ml) was added and the solution was washed with 1 N HCl (3x 30 ml) , 10% $Na_2CO_3$ (3 x 30 ml), water (3 x 30 ml) and dried one $Na_2SO_4$. The product obtained after evaporation of the ethyl acetate was purified by crystallization or flash chromotography. The product was dissolved in 2 ml methanol, flushed with nitrogen for 15-30 minutes and treated with dilute sodium hydroxide for 30-90 minutes. The resulting deprotected thiol was precipitated by adding water, acidified with acetic acid, and the product recovered by lyophilization. The formula of the product is

$HSCH_2CH[CH_2CH(CH_3)_2]CO$-cyclohexylalanine-Ala-$NH_2$

Using the procedure described hereinabove, and the hydrochloride of the appropriate amino acid or depeptide amide, the following compounds were also prepared:

$HSCH_2CH[CH_2CH(CH_3)_2]CO$-Phe-D-Ala-$NH_2$
$HSCH_2CH[CH_2CH(CH_3)_2]CO$-pClPhe-Ala-$NH_2$
$HSCH_2CH[CH_2CH(CH_3)_2]CO$-$N(CH_3)$-Trp-$NH_2$

EXAMPLE 6

Using the procedure of Example 4, herein, the collegenase activity for the compounds prepared in Example 5 was tested, giving the following results:

TABLE II

| | Approximate $IC_{50}$ ( M) |
|---|---|
| $HSCH_2CH[CH_2CH(CH_3)_2]CO$-cyclohexylalanine-Ala-$NH_2$ | 4 |
| $HSCH_2CH[CH_2CH(CH_3)_2]CO$-Phe-D-Ala-$NH_2$ | 3 |
| $HSCH_2CH[CH_2CH(CH_3)_2]CO$-pClPhe-Ala-$NH_2$ | 0.5 |
| $HSCH_2CH[CH_2CH(CH_3)_2]CO$-$N(CH_3)$-Trp-$NH_2$ | 1-10 |

**Claims**

**1.** A compound of the formula:

$R_1SCH(R_2)CH(R_3)CO-AA_1[AA_2]_m[AA_3]_n-X$

wherein m is the integer 0 or 1; n is an integer from 0-2;

$AA_1$ is an amino acid selected from cyclohexylalanine, phenylalanine, naphthylalanine, tryptophan and tyrosine;

$AA_2$ is an amino acid selected from alanine, glycine, leucine, isoleucine and phenylalanine;

$AA_3$ is any amino acid;

$R_1$ is hydrogen, alkyl having from 1-10 carbon atoms, alkanoyl having from 2-10 carbon atoms, or aroyl having from 7-10 carbon atoms;

$R_2$ is hydrogen or alkyl having from 1-6 carbon atoms;

$R_3$ is hydrogen, alkyl having from 2-10 carbon atoms, cycloalkyl having from 3-6 carbon atoms, aryl or arylalkyl, wherein aryl moieties have from 6-10 carbon atoms;

X is $NH_2$, OH, $OCH_3$ or $OCH_2CH_3$;

and salts thereof,

with the proviso that if m is 0 and n is 1, then $AA_3$ is an amino acid selected from alanine, glycine, leucine, isoleucine and phenylalanine,

with the further proviso that if m is 0 and n is 2, then the amino acid $AA_3$ directly bonded to $AA_1$ is selected from alanine, glycine, leucine, isoleucine and phenylalanine, and

with the further proviso that if m and n are both 0, $R_2$ is H and $AA_1$ is phenylalanine, tryptophan or tyrosine, then X is not -OH.

**2.** The compounds of claim 1 wherein $AA_1$ is unsubstituted natural amino acid or mono-substituted with halide or alkyl containing 1 to 6 carbon atoms.

**3.** The compound of claim 1 wherein $R_2$ is hydrogen or $CH_3$, $R_3$ is isobutyl, $R_1$ is hydrogen and X is $NH_2$ or $OCH_2CH_3$.

**4.** The compound of claim 1 wherein m is 1 and $AA_2$ is alanine.

**5.** The compound of claim 3 wherein m is 1 and $AA_2$ is alanine.

**6.** The compound of Claim 5 wherein n is 1 and $AA_3$ is arginine.

**7.** The compound of Claim 3 which has the formula $HSCH_2CH[CH_2CH(CH_3)_2]CO$-Phe-$NH_2$.

**8.** The compound of Claim 3 which has the formula $HSCH_2CH[CH_2CH(CH_3)_2]CO$-Trp-$NH_2$.

**9.** The compound of Claim 3 which has the formula $HSCH_2CH[CH_2CH(CH_3)_2]CO$-Phe-Ala-$NH_2$.

**10.** The compound of Claim 3 which has the formula $HSCH_2CH[CH_2CH(CH_3)_2]CO$-Trp-Ala-$NH_2$.

**11.** The compound of Claim 3 which has the formula $HSCH_2CH[CH_2CH(CH_3)_2]CO$-Nal-$NH_2$.

**12.** The compound of Claim 3 which has the formula $HSCH_2CH[CH_2CH(CH_3)_2]CO$-Nal-Ala-$NH_2$.

**13.** The compound of Claim 3 which has the formula $HSCH_2CH[CH_2CH(CH_3)_2]CO$-Phe-Leu-$NH_2$.

**14.** The compound of Claim 3 which has the formula $HSCH_2CH[CH_2CH(CH_3)_2]CO$-Phe-Phe-$NH_2$.

**15.** The compound of Claim 3 which has the formula $HSCH_2CH[CH_2CH(CH_3)_2]CO$-Phe-Ala-Arg-$NH_2$.

**16.** The compound of Claim 3 which has the formula $HSCH_2CH[CH_2CH(CH_3)_2]CO$-Trp-Ala-Arg-$NH_2$.

**17.** The compound of Claim 3 which has the formula $HSCH_2CH[CH_2CH(CH_3)_2]CO$-Nal-Ala-Arg-$NH_2$.

**18.** The compound of Claim 3 having the formula $HSCH_2CH[CH_2CH(CH_3)_2]CO$-cyclohexylalanine-Ala-$NH_2$.

**19.** The compound of Claim 3 having the formula $HSCH_2CH[CH_2CH(CH_3)_2]CO$-Phe-D-Ala-$NH_2$.

**20.** The compound of Claim 3 having the formula $HSCH_2CH[CH_2CH(CH_3)_2]CO$-pCLPhe-Ala-$NH_2$.

**21.** The compound of Claim 3 having the formula $HSCH_2CH[CH_2CH(CH_3)_2]CO$-N($CH_3$)-Trp-$NH_2$.

**22.** The pharmaceutical composition for treatment of collagenase-related disorders which comprises an effective amount of at least one compound having the formula:

$R_1SCH(R_2)CH(R_3)CO-AA_1[AA_2]_m[AA_3]_n-X$

wherein m is the integer 0 or 1; n is an integer from 0-2;

$AA_1$ is an amino acid selected from phenylalanine, naphthylalanine, tryptophan, tyrosine and cyclohexylalanine;

$AA_2$ is an amino acid selected from alanine, glycine, leucine, isoleucine and phenylalanine;

$AA_3$ is any amino acid;

$R_1$ is hydrogen, alkyl having from 1-10 carbon atoms, alkanoyl having from 2-10 carbon atoms, or aroyl having from 7-10 carbon atoms;

$R_2$ is hydrogen or alkyl having from 1-6 carbon atoms;

$R_3$ is hydrogen, alkyl having from 2-10 carbon atoms, cycloalkyl having from 3-6 carbon atoms, aryl or arylalkyl, wherein aryl moieties have from 6-10 carbon atoms;

X is $NH_2$, OH, $OCH_3$ or $OCH_2CH_3$;

and salts thereof,

with the proviso that if m is 0 and n is 1, then $AA_3$ is an amino acid selected from alanine, glycine, leucine, isoleucine and phenylalanine,

with the further proviso that if m is 0 and n is 2, then the amino acid $AA_3$ directly bonded to $AA_1$ is selected from alanine, glycine, leucine, isoleucine and phenylalanine, and

with the further proviso that if m and n are both 0, $R_2$ is H and $AA_1$ is phenylalanine, tryptophan or tyrosine, then X is not -OH.

**23.** The composition of Claim 22 wherein $AA_1$ is unsubstituted natural amino acid or mono-substituted with alkyl containing 1 to 6 carbon atoms or halogen.

**24.** The composition of Claim 22 wherein $R_2$ is hydrogen or $CH_3$, $R_2$ is isobutyl, $R_1$ is hydrogen and X is $NH_2$.

**25.** The composition of Claim 22 wherein m is 1 and $AA_2$ is alanine.

**26.** The composition of Claim 24 wherein m is 1 and $AA_2$ is alanine.

**27.** The composition of Claim 26 wherein n is 1 and $AA_3$ is arginine.

**28.** The composition of Claim 24 wherein the compound has the formula $HSCH_2CH[CH_2CH(CH_3)_2]CO$-Phe-$NH_2$.

**29.** The composition of Claim 24 wherein the compound has the formula $HSCH_2CH[CH_2CH(CH_3)_2]CO$-Trp-$NH_2$.

**30.** The composition of Claim 24 wherein the compound has the formula $HSCH_2CH[CH_2CH(CH_3)_2]CO$-Phe-Ala-$NH_2$.

**31.** The composition of Claim 24 wherein the compound has the formula $HSCH_2CH[CH_2CH(CH_3)_2]CO$-Trp-Ala-$NH_2$.

**32.** The composition of Claim 24 wherein the compound has the formula $HSCH_2CH[CH_2CH(CH_3)_2]CO$-Nal-$NH_2$.

**33.** The composition of Claim 24 wherein the compound has the formula $HSCH_2CH[CH_2CH(CH_3)_2]CO$-Nal-Ala-$NH_2$.

**34.** The composition of Claim 24 wherein the compound has the formula $HSCH_2CH[CH_2CH(CH_3)_2]CO$-Phe-Leu-$NH_2$.

**35.** The composition of claim 24 wherein the compound has the formula $HSCH_2CH[CH_2CH(CH_3)_2]CO$-Phe-Phe-$NH_2$.

**36.** The composition of Claim 24 wherein the compound has the formula $HSCH_2CH[CH_2CH(CH_3)_2]CO$-Phe-Ala-Arg-$NH_2$.

**37.** The composition of Claim 24 wherein the compound has the formula $HSCH_2CH[CH_2CH(CH_3)_2]CO$-Trp-Ala-Arg-$NH_2$.

**38.** The composition of Claim 24 wherein the compound has the formula $HSCH_2CH[CH_2CH(CH_3)_2]CO$-Nal-Ala-Arg-$NH_2$.

**39.** The composition of Claim 24 wherein the compound has the formula $HSCH_2CH[CH_2CH(CH_3)_2]CO$-cyclohexylalanine-Ala-$NH_2$.

**40.** The composition of Claim 24 wherein the compound has the formula $HSCH_2CH[CH_2CH(CH_3)_2]CO$-Phe-D-Ala-$NH_2$.

**41.** The composition of Claim 24 wherein the compound has the formula $HSCH_2CH[CH_2CH(CH_3)_2]CO$-pCLPhe-Ala-$NH_2$.

**42.** The composition of Claim 24 wherein the compound has the formula $HSCH_2CH[CH_2CH(CH_3)_2]CO$-N-$(CH_3)$-Trp-$NH_2$.

**43.** The use of a compound having a formula

$R_1SCH(R_2)CH(R_3)CO\text{-}AA_1[AA_2]_m[AA_3]_n\text{-}X$

wherein m is the integer 0 or 1; n is an integer from 0-2;
$AA_1$ is an amino acid selected from cyclohexylalanine, phenylalanine, naphthylalanine, tryptophan and tyrosine;
$AA_2$ is an amino acid selected from alanine, glycine, leucine, isoleucine and phenylalanine;
$AA_3$ is any amino acid;
$R_1$ is hydrogen, alkyl having from 1-10 carbon atoms, alkanoyl having from 2-10 carbon atoms, or aroyl having from 7-10 carbon atoms;
$R_2$ is hydrogen or alkyl having from 1-6 carbon atoms;
$R_3$ is hydrogen, alkyl having from 2-10 carbon atoms, cycloalkyl having from 3-6 carbon atoms, aryl or arylalkyl, wherein aryl moieties have from 6-10 carbon atoms;
X is $NH_2$, OH, $OCH_3$ or $OCH_2CH_3$;
and salts thereof,
with the proviso that if m is 0 and n is 1, then $AA_3$ is an amino acid selected from alanine, glycine, leucine, isoleucine and phenylalanine, and
with the further proviso that if m is 0 and n is 2, then the amino acid $AA_3$ directly bonded to $AA_1$ is selected from alanine, glycine, leucine, isoleucine and phenylalanine,
for the manufacture of a medicament for the treatment of mammalian collagenase-related disorders.

## Patentansprüche

**1.** Verbindung der Formel:

$R_1SCH(R_2)CH(R_3)CO\text{-}AA_1[AA_2]_m[AA_3]_n\text{-}X$

worin m die Zahl 0 oder 1 ist; n eine Ganzzahl von 0 bis 2 ist ;
$AA_1$ eine Aminosäure ist, ausgewählt aus Cyclohexylalanin, Phenylalanin, Naphthylalanin, Tryptophan und Tyrosin;
$AA_2$ eine Aminosäure ist, ausgewählt aus Alanin, Glycin, Leucin, Isoleucin und Phenylalanin;
$AA_3$ eine beliebige Aminosäure ist;
$R_1$ Wasserstoff, Alkyl mit 1 bis 10 Kohlenstoffatomen, Alkanoyl mit 2 bis 10 Kohlenstoffatomen oder Aroyl mit 7 bis 10 Kohlenstoffatomen ist;
$R_2$ Wasserstoff oder Alkyl mit 1 bis 6 Kohlenstoffatomen ist;

$R_3$ Wasserstoff, Alkyl mit 2 bis 10 Kohlenstoffatomen, Cycloalkyl mit 3 bis 6 Kohlenstoffatomen, Aryl oder Arylalkyl ist, wobei die Aryleinheiten 6 bis 10 Kohlenstoffatome enhalten;

X $NH_2$, OH, $OCH_3$, oder $OCH_2CH_3$ ist;

und deren Salze,

mit der Maßgabe, daß wenn m Null und n 1 ist, dann ist $AA_3$ eine Aminosäure,ausgewählt aus Alanin, Glycin, Leucin, Isoleucin und Phenylalanin,

mit der weiteren Maßgabe, daß wenn m Null und n 2 ist, dann ist die Aminosäure $AA_3$, die direkt an $AA_1$ gebunden ist, ausgewählt aus Alanin, Glycin, Leucin, Isoleucin und Phenylalanin, und

mit der weiteren Maßgabe, daß wenn m und n beide Null sind, $R_2$ H ist und $AA_1$ Phenylalanin, Tryptophan oder Tyrosin ist, dann ist X nicht -OH.

2. Verbindung gemäß Anspruch 1, dadurch **gekennzeichnet,** daß $AA_1$ eine unsubstituierte natürliche Aminosäure oder mit einem Halogenid oder einer Alkylgruppe mit 1 bis 6 Kohlenstoffatomen monosubstituiert ist.

3. Verbindung gemäß Anspruch 1, dadurch **gekennzeichnet,** daß $R_2$ Wasserstoff oder $CH_3$ ist, $R_3$ Isobutyl ist, $R_1$ Wasserstoff ist und X $NH_2$ oder $OCH_2CH_3$ist.

4. Verbindung gemäß Anspruch 1, dadurch **gekennzeichnet,** daß m 1 und $AA_2$ Alanin ist.

5. Verbindung gemäß Anspruch 3, dadurch **gekennzeichnet,** daß m 1 und $AA_2$ Alanin ist.

6. Verbindung gemäß Anspruch 5, dadurch **gekennzeichnet,** daß n 1 und $AA_3$ Arginin ist.

7. Verbindung gemäß Anspruch 3 mit der Formel $HSCH_2CH[CH_2CH(CH_3)_2]CO$-Phe-$NH_2$.

8. Verbindung gemäß Anspruch 3 mit der Formel $HSCH_2CH[CH_2CH(CH_3)_2]CO$-Trp-$NH_2$.

9. Verbindung gemäß Anspruch 3 mit der Formel $HSCH_2CH[CH_2CH(CH_3)_2]CO$-Phe-Ala-$NH_2$.

10. Verbindung gemäß Anspruch 3 mit der Formel $HSCH_2CH[CH_2CH(CH_3)_2]CO$-Trp-Ala-$NH_2$.

11. Verbindung gemäß Anspruch 3 mit der Formel $HSCH_2CH[CH_2CH(CH_3)_2]CO$-Nal-$NH_2$.

12. Verbindung gemäß Anspruch 3 mit der Formel $HSCH_2CH[CH_2CH(CH_3)_2]CO$-Nal-Ala-$NH_2$.

13. Verbindung gemäß Anspruch 3 mit der Formel $HSCH_2CH[CH_2CH(CH_3)_2]CO$-Phe-Leu-$NH_2$.

14. Verbindung gemäß Anspruch 3 mit der Formel $HSCH_2CH[CH_2CH(CH_3)_2]CO$-Phe-Phe-$NH_2$.

15. Verbindung gemäß Anspruch 3 mit der Formel $HSCH_2CH[CH_2CH(CH_3)_2]CO$-Phe-Ala-Arg-$NH_2$.

16. Verbindung gemäß Anspruch 3 mit der Formel $HSCH_2CH[CH_2CH(CH_3)_2]CO$-Trp-Ala-Arg-$NH_2$.

17. Verbindung gemäß Anspruch 3 mit der Formel $HSCH_2CH[CH_2CH(CH_3)_2]CO$-Nal-Ala-Arg-$NH_2$.

18. Verbindung gemäß Anspruch 3 mit der Formel $HSCH_2CH[CH_2CH(CH_3)_2]CO$-cyclohexylalanin-Ala-$NH_2$.

19. Verbindung gemäß Anspruch 3 mit der Formel $HSCH_2CH[CH_2CH(CH_3)_2]CO$-Phe-D-Ala-$NH_2$.

20. Verbindung gemäß Anspruch 3 mit der Formel $HSCH_2CH[CH_2CH(CH_3)_2]CO$-pClPhe-Ala-$NH_2$.

21. Verbindung gemäß Anspruch 3 mit der Formel $HSCH_2CH[CH_2CH(CH_3)_2]CO$-$N(CH_3)$-Trp-$NH_2$.

22. Pharmazeutische Zusammensetzung zur Behandlung von mit Collagenase zusammenhängenden Störungen, umfassend eine wirksame Menge mindestens einer Verbindung der Formel:

$R_1SCH(R_2)CH(R_3)CO-AA_1[AA_2]_m[AA_3]_n-X$

worin m die Zahl 0 oder 1 ist; n eine Ganzzahl von 0 bis 2 ist ;

$AA_1$ eine Aminosäure ist, ausgewählt aus Cyclohexylalanin, Phenylalanin, Naphthylalanin, Tryptophan und Tyrosin;

$AA_2$ eine Aminosäure ist, ausgewählt aus Alanin, Glycin, Leucin, Isoleucin und Phenylalanin;

$AA_3$ eine beliebige Aminosäure ist;

$R_1$ Wasserstoff, Alkyl mit 1 bis 10 Kohlenstoffatomen, Alkanoyl mit 2 bis 10 Kohlenstoffatomen oder Aroyl mit 7 bis 10 Kohlenstoffatomen ist;

$R_2$ Wasserstoff oder Alkyl mit 1 bis 6 Kohlenstoffatomen ist;

$R_3$ Wasserstoff, Alkyl mit 2 bis 10 Kohlenstoffatomen, Cycloalkyl mit 3 bis 6 Kohlenstoffatomen, Aryl oder Arylalkyl ist, wobei die Aryleinheiten 6 bis 10 Kohlenstoffatome enhalten;

X $NH_2$, OH, $OCH_3$, oder $OCH_2CH_3$ ist;

und deren Salze,

mit der Maßgabe, daß wenn m Null und n 1 ist, dann ist $AA_3$ eine Aminosäure,ausgewählt aus Alanin, Glycin, Leucin, Isoleucin und Phenylalanin,

mit der weiteren Maßgabe, daß wenn m Null und n 2 ist, dann ist die Aminosäure $AA_3$, die direkt an $AA_1$ gebunden ist, ausgewählt aus Alanin, Glycin, Leucin, Isoleucin und Phenylalanin, und

mit der weiteren Maßgabe, daß wenn m und n beide Null sind, $R_2$ H ist und $AA_1$ Phenylalanin, Tryptophan oder Tyrosin ist, dann ist X nicht -OH.

**23.** Verbindung gemäß Anspruch 22, dadurch **gekennzeichnet,** daß $AA_1$ eine unsubstituierte natürliche Aminosäure oder monosubstituiert mit einer Alkylgruppe mit 1 bis 6 Kohlenstoffatomen oder Halogen ist.

**24.** Zusammensetzung gemäß Anspruch 22, dadurch **gekennzeichnet,** daß $R_2$ Wasserstoff oder $CH_3$ ist, $R_3$ Isobutyl ist, $R_1$ Wasserstoff ist und X $NH_2$ ist.

**25.** Zusammensetzung gemäß Anspruch 22, dadurch **gekennzeichnet,** daß m 1 und $AA_2$ Alanin ist.

**26.** Zusammensetzung gemäß Anspruch 24, dadurch **gekennzeichnet,** daß m 1 und $AA_2$ Alanin ist.

**27.** Zusammensetzung gemäß Anspruch 26, dadurch **gekennzeichnet,** daß n 1 und $AA_3$ Arginin ist.

**28.** Zusammensetzung gemäß Anspruch 24, dadurch **gekennzeichnet,** daß die Verbindung die Formel $HSCH_2CH[CH_2CH(CH_3)_2]CO$-Phe-$NH_2$ hat.

**29.** Zusammensetzung gemäß Anspruch 24, dadurch **gekennzeichnet,** daß die Verbindung die Formel $HSCH_2CH[CH_2CH(CH_3)_2]CO$-Trp-$NH_2$ hat.

**30.** Zusammensetzung gemäß Anspruch 24, dadurch **gekennzeichnet,** daß die Verbindung die Formel $HSCH_2CH[CH_2CH(CH_3)_2]CO$-Phe-Ala-$NH_2$ hat.

**31.** Zusammensetzung gemäß Anspruch 24, dadurch **gekennzeichnet,** daß die Verbindung die Formel $HSCH_2CH[CH_2CH(CH_3)_2]CO$-Trp-Ala-$NH_2$ hat.

**32.** Zusammensetzung gemäß Anspruch 24, dadurch **gekennzeichnet,** daß die Verbindung die Formel $HSCH_2CH[CH_2CH(CH_3)_2]CO$-Nal-$NH_2$ hat.

**33.** Zusammensetzung gemäß Anspruch 24, dadurch **gekennzeichnet,** daß die Verbindung die Formel $HSCH_2CH[CH_2CH(CH_3)_2]CO$-Nal-Ala-$NH_2$ hat.

**34.** Zusammensetzung gemäß Anspruch 24, dadurch **gekennzeichnet,** daß die Verbindung die Formel $HSCH_2CH[CH_2CH(CH_3)_2]CO$-Phe-Leu-$NH_2$ hat.

**35.** Zusammensetzung gemäß Anspruch 24, dadurch **gekennzeichnet,** daß die Verbindung die Formel $HSCH_2CH[CH_2CH(CH_3)_2]CO$-Phe-Phe-$NH_2$.

**36.** Zusammensetzung gemäß Anspruch 24, dadurch **gekennzeichnet,** daß die Verbindung die Formel $HSCH_2CH[CH_2CH(CH_3)_2]CO$-Phe-Ala-Arg-$NH_2$ hat.

**37.** Zusammensetzung gemäß Anspruch 24, dadurch **gekennzeichnet,** daß die Verbindung die Formel $HSCH_2CH[CH_2CH(CH_3)_2]CO$-Trp-Ala-Arg-$NH_2$ hat.

**38.** Zusammensetzung gemäß Anspruch 24, dadurch **gekennzeichnet,** daß die Verbindung die Formel $HSCH_2CH[CH_2CH(CH_3)_2]CO$-Nal-Ala-Arg-$NH_2$ hat.

**39.** Zusammensetzung gemäß Anspruch 24, dadurch **gekennzeichnet,** daß die Verbindung die Formel $HSCH_2CH[CH_2CH(CH_3)_2]CO$-cyclohexylalanin-Ala-$NH_2$ hat.

**40.** Zusammensetzung gemäß Anspruch 24, dadurch **gekennzeichnet,** daß die Verbindung die Formel $HSCH_2CH[CH_2CH(CH_3)_2]CO$-Phe-D-Ala-$NH_2$ hat.

**41.** Zusammensetzung gemäß Anspruch 24, dadurch **gekennzeichnet**, daß die Verbindung die Formel $HSCH_2CH[CH_2CH(CH_3)_2]CO$-pClPhe-Ala-$NH_2$ hat.

**42.** Zusammensetzung gemäß Anspruch 24, dadurch **gekennzeichnet,** daß die Verbindung die Formel $HSCH_2CH[CH_2CH(CH_3)_2]CO$-$N(CH_3)$-Trp-$NH_2$.

**43.** Verwendung einer Verbindung der Formel

$R_1SCH(R_2)CH(R_3)CO$-$AA_1[AA_2]_m[AA_3]_n$-X

worin m die Zahl 0 oder 1 ist; n eine Ganzzahl von 0 bis 2 ist;
$AA_1$ eine Aminosäure, ausgewählt aus Cyclohexylalanin, Phenylalanin, Naphthylalanin, Tryptophan und Tyrosin, ist;
$AA_2$ eine Aminosäure, ausgewählt aus Alanin, Glycin, Leucin, Isoleucin und Phenylalanin, ist ;
$AA_3$ eine beliebige Aminosäure ist;
$R_1$ Wasserstoff, Alkyl mit 1 bis 10 Kohlenstoffatomen, Alkanoyl mit 2 bis 10 Kohlenstoffatomen oder Aroyl mit 7 bis 10 Kohlenstoffatomen ist;
$R_2$ Wasserstoff oder Alkyl mit 1 bis 6 Kohlenstoffatomen ist;
$R_3$ Wasserstoff, Alkyl mit 2 bis 10 Kohlenstoffatomen, Cycloalkyl mit 3 bis 6 Kohlenstoffatomen, Aryl oder Arylalkyl ist, wobei die Aryleinheiten 6 bis 10 Kohlenstoffatome enhalten;
X $NH_2$, OH, $OCH_3$, oder $OCH_2CH_3$ ist;
und deren Salze,
mit der Maßgabe, daß wenn m Null und n 1 ist, dann ist $AA_3$ eine Aminosäure, ausgewählt aus Alanin, Glycin, Leucin, Isoleucin und Phenylalanin, und
mit der weiteren Maßgabe, daß wenn m Null und n 2 ist, dann ist die Aminosäure $AA_3$, die direkt an $AA_1$ gebunden ist, ausgewählt aus Alanin, Glycin, Leucin, Isoleucin und Phenylalanin,
zur Herstellung eines Medikaments zur Behandlung von mit Säugetiercollagenase zusammenhängenden Störungen.

## Revendications

**1.** Composé répondant à la formule :

$R_1SCH(R_2)CH(R_3)CO$-$AA_1[AA_2]_m[AA_3]_n$ - X

dans laquelle m est un entier égal à 0 ou 1 ; n est un entier de 0 à 2 ;
$AA_1$ est un amino acide choisi parmi la cyclohexylalanine, la phénylalanine, la naphtylalanine, le tryptophane et la tyrosine ;
$AA_2$ est un amino acide choisi parmi l'alanine, la glycine, la leucine, l'isoleucine et la phénylalanine ;
$AA_3$ est n'importe quel amino acide ;
$R_1$ est un atome d'hydrogène, un groupe alkyle en $C_1$ à $C_{10}$, alcanoyle en $C_2$ à $C_{10}$ ou aroyle en $C_7$ à $C_{10}$ ;

$R_2$ est un atome d'hydrogène ou un groupe alkyle en $C_1$ à $C_6$ ;

$R_3$ est un atome d'hydrogène, un groupe alkyle en $C_2$ à $C_{10}$, cycloalkyle en $C_3$ à $C_6$, aryle ou arylalkyle, dans lequel les parties aryle ont de 6 à 10 atomes de carbone ;

X est $NH_2$, OH, $OCH_3$ ou $OCH_2CH_3$ ;

et leurs sels,

sous réserve que, si m est 0 et n est 1, $AA_3$ est un amino acide choisi parmi l'alanine, la glycine, la leucine, l'isoleucine et la phénylalanine,

sous réserve en outre que, si m est 0 et n est 2, l'amino acide $AA_3$ directement lié à $AA_1$ est choisi parmi l'alanine, la glycine, la leucine, l'isoleucine et la phénylalanine ; et

sous cette réserve supplémentaire que, si m et n sont tous deux 0, $R_2$ est H et $AA_1$ est la phénylalanine, le tryptophane ou la tyrosine, X n'est pas -OH.

**2.** Composés selon la revendication 1, dans lesquels $AA_1$ est un amino acide naturel non substitué ou monosubstitué par un halogénure ou un groupe alkyle en $C_1$ à $C_6$.

**3.** Composé selon la revendication 1, dans lequel $R_2$ est un atome d'hydrogène ou $CH_3$, $R_3$ est un groupe isobutyle, $R_1$ est un atome d'hydrogène et X est $NH_2$ ou $OCH_2CH_3$.

**4.** Composé selon la revendication 1, dans lequel m est 1 et $AA_2$ est l'alanine.

**5.** Composé selon la revendication 3, dans lequel m est 1 et $AA_2$ est l'alanine.

**6.** Composé selon la revendication 5, dans lequel n est 1 et $AA_3$ est l'arginine.

**7.** Composé selon la revendication 3, répondant à la formule $HSCH_2CH[CH_2CH(CH_3)_2]CO$-Phe-$NH_2$.

**8.** Composé selon la revendication 3, répondant à la formule $HSCH_2CH[CH_2CH(CH_3)_2]CO$-Trp-$NH_2$.

**9.** Composé selon la revendication 3, répondant à la formule $HSCH_2CH[CH_2CH(CH_3)_2]CO$-Phe-Ala-$NH_2$.

**10.** Composé selon la revendication 3, répondant à la formule $HSCH_2CH[CH_2CH(CH_3)_2]CO$-Trp-Ala-$NH_2$.

**11.** Composé selon la revendication 3, répondant à la formule $HSCH_2CH[CH_2CH(CH_3)_2]CO$-Nal-$NH_2$.

**12.** Composé selon la revendication 3, répondant à la formule $HSCH_2CH[CH_2CH(CH_3)_2]CO$-Nal-Ala-$NH_2$.

**13.** Composé selon la revendication 3, répondant à la formule $HSCH_2CH[CH_2CH(CH_3)_2]CO$-Phe-Leu-$NH_2$.

**14.** Composé selon la revendication 3, répondant à la formule $HSCH_2CH[CH_2CH(CH_3)_2]CO$-Phe-Phe-$NH_2$.

**15.** Composé selon la revendication 3, répondant à la formule $HSCH_2CH[CH_2CH(CH_3)_2]CO$-Phe-Ala-Arg-$NH_2$.

**16.** Composé selon la revendication 3, répondant à la formule $HSCH_2CH[CH_2CH(CH_3)_2]CO$-Trp-Ala-Arg-$NH_2$.

**17.** Composé selon la revendication 3, répondant à la formule $HSCH_2CH[CH_2CH(CH_3)_2]CO$-Nal-Ala-Arg-$NH_2$.

**18.** Composé selon la revendication 3, répondant à la formule $HSCH_2CH[CH_2CH(CH_3)_2]CO$-cyclohexylalanine-Ala-$NH_2$.

**19.** Composé selon la revendication 3, répondant à la formule $HSCH_2CH[CH_2CH(CH_3)_2]CO$-Phe-D-Ala-$NH_2$.

**20.** Composé selon la revendication 3, répondant à la formule $HSCH_2CH[CH_2CH(CH_3)_2]CO$-pCLPhe-Ala-$NH_2$.

**21.** Composé selon la revendication 3, répondant à la formule $HSCH_2CH[CH_2CH(CH_3)_2]CO-N(CH_3)$-Trp-$NH_2$.

**22.** Composition pharmaceutique pour le traitement des troubles liés à la collagénase, qui comprend une quantité efficace d'au moins un composé répondant à la formule :

$R_1SCH(R_2)CH(R_3)CO-AA_1[AA_2]_m[AA_3]_n - X$

dans laquelle m est un entier égal à 0 ou 1 ; n est un entier de 0 à 2 ;

$AA_1$ est un amino acide choisi parmi la cyclohexylalanine, la phénylalanine, la naphtylalanine, le tryptophane et la tyrosine ;

$AA_2$ est un amino acide choisi parmi l'alanine, la glycine, la leucine, l'isoleucine et la phénylalanine ;

$AA_3$ est n'importe quel amino acide ;

$R_1$ est un atome d'hydrogène, un groupe alkyle en $C_1$ à $C_{10}$, alcanoyle en $C_2$ à $C_{10}$ ou aroyle en $C_7$ à $C_{10}$ ;

$R_2$ est un atome d'hydrogène ou un groupe alkyle en $C_1$ à $C_6$ ;

$R_3$ est un atome d'hydrogène, un groupe alkyle en $C_2$ à $C_{10}$, cycloalkyle en $C_3$ à $C_6$, aryle ou arylalkyle, dans lequel les parties aryle ont de 6 à 10 atomes de carbone ;

X est $NH_2$, OH, $OCH_3$ ou $OCH_2CH_3$ ;

et leurs sels,

sous réserve que, si m est 0 et n est 1, $AA_3$ est un amino acide choisi parmi l'alanine, la glycine, la leucine, l'isoleucine et la phénylalanine,

sous réserve en outre que, si m est 0 et n est 2, l'amino acide $AA_3$ directement lié à $AA_1$ est choisi parmi l'alanine, la glycine, la leucine, l'isoleucine et la phénylalanine ; et

sous cette réserve supplémentaire que, si m et n sont tous deux 0, $R_2$ est H et $AA_1$ est la phénylalanine, le tryptophane ou la tyrosine, X n'est pas -OH.

**23.** Composition selon la revendication 22, dans laquelle $AA_1$ est un amino acide naturel non substitué ou mono-substitué par un halogénure ou un groupe alkyle en $C_1$ à $C_6$.

**24.** Composition selon la revendication 22, dans laquelle $R_2$ est un atome d'hydrogène ou $CH_3$, $R_3$ est un groupe isobutyle, $R_1$ est un atome d'hydrogène et X est $NH_2$.

**25.** Composition selon la revendication 22, dans laquelle m est 1 et $AA_2$ est l'alanine.

**26.** Composition selon la revendication 24, dans laquelle m est 1 et $AA_2$ est l'alanine.

**27.** Composition selon la revendication 26, dans laquelle n est 1 et $AA_3$ est l'arginine.

**28.** Composition selon la revendication 24, dans laquelle le composé répond à la formule $HSCH_2CH$-$[CH_2CH(CH_3)_2]$CO-Phe-$NH_2$.

**29.** Composition selon la revendication 24, dans laquelle le composé répond à la formule $HSCH_2CH$-$[CH_2CH(CH_3)_2]$CO-Trp-$NH_2$.

**30.** Composition selon la revendication 24, dans laquelle le composé répond à la formule $HSCH_2CH$-$[CH_2CH(CH_3)_2]$CO-Phe-Ala-$NH_2$.

**31.** Composition selon la revendication 24, dans laquelle le composé répond à la formule $HSCH_2CH$-$[CH_2CH(CH_3)_2]$CO-Trp-Ala-$NH_2$.

**32.** Composition selon la revendication 24, dans laquelle le composé répond à la formule $HSCH_2CH$-$[CH_2CH(CH_3)_2]$CO-Nal-$NH_2$.

**33.** Composition selon la revendication 24, dans laquelle le composé répond à la formule $HSCH_2CH$-$[CH_2CH(CH_3)_2]$CO-Nal-Ala-$NH_2$.

**34.** Composition selon la revendication 24, dans laquelle le composé répond à la formule $HSCH_2CH$-[$CH_2CH(CH_3)_2$]CO-Phe-Leu-$NH_2$.

**35.** Composition selon la revendication 24, dans laquelle le composé répond à la formule $HSCH_2CH$-[$CH_2CH(CH_3)_2$]CO-Phe-Phe-$NH_2$.

**36.** Composition selon la revendication 24, dans laquelle le composé répond à la formule $HSCH_2CH$-[$CH_2CH(CH_3)_2$]CO-Phe-Ala-Arg-$NH_2$.

**37.** Composition selon la revendication 24, dans laquelle le composé répond à la formule $HSCH_2CH$-[$CH_2CH(CH_3)_2$]CO-Trp-Ala-Arg-$NH_2$.

**38.** Composition selon la revendication 24, dans laquelle le composé répond à la formule $HSCH_2CH$-[$CH_2CH(CH_3)_2$]CO-Nal-Ala-Arg-$NH_2$.

**39.** Composition selon la revendication 24, dans laquelle le composé répond à la formule $HSCH_2CH$-[$CH_2CH(CH_3)_2$]CO-cyclohexylalanine-Ala-$NH_2$.

**40.** Composition selon la revendication 24, dans laquelle le composé répond à la formule $HSCH_2CH$-[$CH_2CH(CH_3)_2$]CO-Phe-D-Ala-$NH_2$.

**41.** Composition selon la revendication 24, dans laquelle le composé répond à la formule $HSCH_2CH$-[$CH_2CH(CH_3)_2$]CO-pCLPhe-Ala-$NH_2$.

**42.** Composition selon la revendication 24, dans laquelle le composé répond à la formule $HSCH_2CH$-[$CH_2CH(CH_3)_2$]CO-N($CH_3$)-Trp-$NH_2$.

**43.** Utilisation d'un composé répondant à la formule :

$R_1SCH(R_2)CH(R_3)CO\text{-}AA_1[AA_2]_m[AA_3]_n$ - X

dans laquelle m est un entier égal à 0 ou 1 ; n est un entier de 0 à 2 ;

$AA_1$ est un amino acide choisi parmi la cyclohexylalanine, la phénylalanine, la naphtylalanine, le tryptophane et la tyrosine ;

$AA_2$ est un amino acide choisi parmi l'alanine, la glycine, la leucine, l'isoleucine et la phénylalanine ;

$AA_3$ est n'importe quel amino acide ;

$R_1$ est un atome d'hydrogène, un groupe alkyle en $C_1$ à $C_{10}$, alcanoyle en $C_2$ à $C_{10}$ ou aroyle en $C_7$ à $C_{10}$ ;

$R_2$ est un atome d'hydrogène ou un groupe alkyle en $C_1$ à $C_6$ ;

$R_3$ est un atome d'hydrogène, un groupe alkyle en $C_2$ à $C_{10}$, cycloalkyle en $C_3$ à $C_6$, aryle ou arylalkyle, dans lequel les parties aryle ont de 6 à 10 atomes de carbone ;

X est $NH_2$, OH, $OCH_3$ ou $OCH_2CH_3$ ;

et leurs sels,

sous réserve que, si m est 0 et n est 1, $AA_3$ est un amino acide choisi parmi l'alanine, la glycine, la leucine, l'isoleucine et la phénylalanine, et

sous réserve en outre que, si m est 0 et n est 2, l'amino acide $AA_3$ directement lié à $AA_1$ est choisi parmi l'alanine, la glycine, la leucine, l'isoleucine et la phénylalanine,

pour la fabrication d'un médicament pour le traitement des troubles liés à la collagénase des mammifères.